# EUROPEAN PATENT APPLICATION

(11) **EP 4 517 316 A1**
(43) Date of publication of application: **05.03.2025**
(21) Application number: 23791737.2
(22) Date of filing: 10.04.2023
(51) Int. Cl.: G01N 27/333, G01N 27/28, G01N 27/416, G01N 37/00

(54) **MICRO-ANALYSIS CHIP**

(30) Priority: 23.04.2022 JP 2022071207
(71) Applicant: CANON KABUSHIKI KAISHA, Tokyo 146-8501 (JP)
(72) Inventor: MAEDA Harunobu, Tokyo 146-8501 (JP); YAMAMOTO Takeshi, Tokyo 146-8501 (JP); MIYAZAKI Keiji, Tokyo 146-8501 (JP); FUKATSU Makoto, Tokyo 146-8501 (JP); TANAKA Masanori, Tokyo 146-8501 (JP); MIURA Jun, Tokyo 146-8501 (JP); ENOKIDO Fuka, Tokyo 146-8501 (JP); MATSUKAWA Akihisa, Tokyo 146-8501 (JP); IKAMI Yasukazu, Tokyo 146-8501 (JP); ABE Katsuichi, Tokyo 146-8501 (JP)
(74) Representative: TBK
(86) International application number: PCT/JP2023/014600
(87) International publication number: WO 2023/204088

(57) **Abstract**

In a microanalytical chip made up of a first porous substrate and a second porous substrate laminated on each other, a first channel chamber, a second channel chamber, and a first channel connecting the first channel chamber with the second channel chamber are formed by a channel wall in the first porous substrate, a reference electrode is disposed in the first channel chamber, a working electrode is disposed in the second channel chamber, the second porous substrate is disposed so as to overlap at least part of a region, where the working electrode is formed, on a surface of the first porous substrate.

## Description

### Technical Field

The present invention relates to a microanalytical chip in which a microchannel is formed in a porous substrate.

### Background Art

In recent years, a microanalytical chip that allows an analysis in biochemistry to be efficiently performed in a single chip by using a fine channel with a microsize has become a focus of attention in a wide range of fields. Specifically, a microanalytical chip has become a focus of attention not only in the research of biochemistry but also in the fields of medicine, innovative drug development, health care, environment, food, and the like.

A microanalytical chip in which a fine channel with a micrometer size was formed on glass or silicon by photolithography, with a metal mold, or the like and pretreatment, agitation, mixing, reaction, and detection of a sample were performed on a single chip was developed in the first half of 1990s. As a result, a size reduction of an inspection system, a quick analysis, a reduction of samples and waste liquid, and the like were achieved.

An electrochemical analysis is to measure a potential between electrodes immersed in a sample to be analyzed and is widely used in the fields of medicine, environment, and the like. An existing electrochemical analysis is performed with an advanced instrument by an engineer, so fields and resources for measurement are limited to a certain extent. However, for the purpose of medical care in developing countries, backwoods or disaster sites where medical equipment is not enough and usage at airports or the like where the spread of infectious diseases needs to be checked near water, there are needs for low-cost, easy-to-handle, and disposable microanalytical chips for electrochemical analysis.

NPL 1 suggests a filter paper-based device for measuring the concentration of Na ions and the concentration of K ions. The device is formed on a sheet of filter paper and has a dispensing portion for dispensing a sample. The dispensed sample permeates from the dispensing portion into regions of a working electrode and a reference electrode to electrically connect both electrodes, with the result that measurement of a potential difference is possible. In the device, KCl ionic crystals are deposited on the reference electrode to obtain a stable potential at the reference electrode, and KCl ionic crystals dissolve in the sample at the time of measurement to keep Cl ions in the reference electrode region at a high concentration, with the result that the stable potential of the reference electrode is obtained. Furthermore, only ions to be measured are selected by a K ion selective membrane formed to cover the working electrode, thus making it possible to perform measurement without receiving the influence of other ions.

PTL 1 suggests a microanalytical chip for electrochemical analysis. The device has a two-layer structure. A dispensing portion for dispensing a sample is provided at an upper layer part. The dispensed sample substantially penetrates through the thickness of a porous hydrophilic layer and permeates into an upper surface of an electrode assembly on a lower layer substrate. Then, a plurality of electrodes is electrically connected, and electrochemical measurement is possible.

### Citation List

### Patent Literature

PTL 1: U.S. Patent Application Publication No. 2012/0181184

### Non Patent Literature

NPL 1: Nipapan Ruecha, Orawon Chailapakul, Koji Suzuki and Daniel Chitterio "Fully Inkjet-Printed Paper-Based Potentiometric Ion-Sensing Devices" Analytical Chemistry August 29, 2017 Published, 89, PP. 10608-10616

### Summary of Invention

### Technical Problem

However, with the above-described configuration, an undesirable influence can be exerted on the measurement accuracy of sample concentration. Specifically, an undesirable influence can be exerted on the measurement sensitivity of electrolyte concentration, the ion selectivity of specific ions, potential stability at the time of measurement, or the like. A reason for the above is that a contact area between a sample and an ion selective membrane is insufficient or the contact is unstable.

With the configuration described in NPL 1, to obtain high measurement accuracy, forming conditions of the ion selective membrane need to be accurately controlled. Specifically, the ion selective membrane needs to penetrate into an air gap of the porous substrate so that the contact area between the porous substrate and the ion selective membrane in the porous substrate increases. However, NPL 1 does not describe the details of a method of controlling permeation. When the area of the ion selective membrane is increased to increase the area of a boundary surface between the ion selective membrane and the porous substrate, the size of the channel device increases.

With the configuration described in PTL 1, a porous hydrophilic layer contacts with an upper surface of an electrode assembly to supply a sample. However, a channel through which the sample can permeate is not provided in the substrate that supports the electrode assembly, so a sample flow rate caused by capillarity is low, and a period of time from dispensing to the end of measurement sometimes extends. A sample contact sometimes becomes unstable at a side part of the electrode assembly with no channel, so a measurement potential can be undesirably influenced.

The present disclosure is made in view of the above inconvenience, and it is an object of present disclosure to provide a microanalytical chip capable of quickly obtaining high measurement sensitivity of electrolyte concentration, ion selectivity, and potential stability.

### Solution to Problem

To achieve the above object, one aspect of the present disclosure provides a microanalytical chip made up of a first porous substrate and a second porous substrate laminated on each other, wherein a first channel chamber, a second channel chamber, and a first channel connecting the first channel chamber with the second channel chamber are formed by a channel wall in the first porous substrate, a reference electrode is disposed in the first channel chamber, a working electrode is disposed in the second channel chamber, the second porous substrate is disposed so as to overlap at least part of a region, where the working electrode is formed, on a surface of the first porous substrate.

### Advantageous Effects of Invention

According to one aspect of the present disclosure, it is possible to provide a microanalytical chip excellent in the measurement sensitivity of electrolyte concentration, ion selectivity, and temporally potential stability.

### Brief Description of Drawings

[Fig. 1] Fig. 1 is a schematic sectional view that shows the configuration of a microanalytical chip P1 according to Example 1.
[Fig. 2] Fig. 2 is a view that schematically shows a relative positional relationship and a magnitude relationship among a channel chamber 1, a channel chamber 2, a channel 3, a channel wall 5, a dispensing portion 6, a reference electrode 7, a working electrode 8, an ion selective membrane 9, and an ionic crystal 10 of the microanalytical chip P1 according to Example 1.
[Fig. 3] Fig. 3 is a schematic sectional view that shows the configuration of a microanalytical chip P2 according to Comparative Example 1.
[Fig. 4A] Fig. 4A is an example of measurement results according to Example 1 and Comparative Example 1.
[Fig. 4B] Fig. 4B is an example of measurement results according to Example 1 and Comparative Example 1.
[Fig. 5A] Fig. 5A is an example of measurement results according to Example 1 and Comparative Example 1.
[Fig. 5B] Fig. 5B is an example of measurement results according to Example 1 and Comparative Example 1.
[Fig. 6] Fig. 6 is a schematic sectional view that shows the filtration effect of Example 1.
[Fig. 7] Fig. 7 is a schematic sectional view that shows the filtration effect of Comparative Example 1.
[Fig. 8] Fig. 8 is a schematic sectional view that shows the configuration of a microanalytical chip P3 according to Comparative Example 2.
[Fig. 9] Fig. 9 is a conceptual view that shows temporal changes in the behavior of a dispensed sample according to Example 1 and Comparative Example 1.
[Fig. 10] Fig. 10 is a schematic sectional view that shows the configuration of the microanalytical chip P2 according to Modification 1 of Example 1.

### Description of Embodiments

In the specification, the phrase "greater than or equal to XX and less than or equal to YY" or the phrase "XX to YY" that indicates a numeric range means a numeric range including a lower limit and an upper limit that are end points unless otherwise noted. In a case where a numeric value is described in a stepwise manner, a selected combination of an upper limit and a lower limit of each numeric range is described.

A microanalytical chip according to the present invention to solve the above-described inconvenience will be described with reference to the following embodiments. The embodiments described below are illustrative and are not intended to limit the technical scope of the present invention to those only.

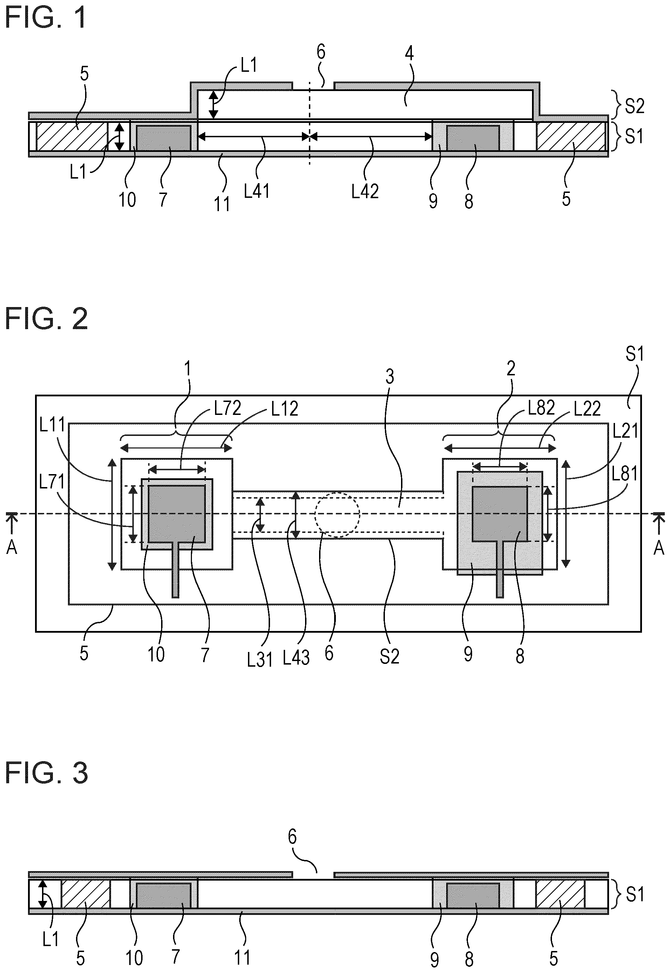

Fig. 1 schematically shows a sectional view of a microanalytical chip P1, taken along the line A-A in Fig. 2.

Fig. 2 is a view that schematically shows a relative positional relationship and a magnitude relationship among a channel chamber 1, a channel chamber 2, a channel 3, a channel wall 5, a dispensing portion 6, a reference electrode 7, a working electrode 8, an ion selective membrane 9, and an ionic crystal 10 of the microanalytical chip P1 according to Example 1.

The microanalytical chip P1 is made up of a porous substrate S1 and a porous substrate S2. The porous substrate S1 and the porous substrate S2 are laminated in a thickness direction.

The porous substrate S1 has a first channel region surrounded by the channel wall 5. A channel pattern in the porous substrate S1 is made up of the channel chamber 1 (first channel chamber), the channel chamber 2 (second channel chamber), and the channel 3. The channel 3 (first channel) connects the channel chamber 1 (first channel chamber) with the channel chamber 2 (second channel chamber).

The reference electrode 7 is disposed in the channel chamber 1. The ionic crystal 10 having a sample solubility is disposed on the surface of the reference electrode 7.

The working electrode 8 is disposed in the channel chamber 2.

The porous substrate S2 includes the dispensing portion 6 for dispensing a sample and a channel 4 (second channel) that connects the dispensing portion 6 with a region in which the working electrode 8 is formed.

The channel 4 is provided so as to overlap at least part of a region on the surface of the porous substrate S1 where the working electrode 8 is formed.

A location connecting at least the dispensing portion 6 with the working electrode 8 on the upper surface of the porous substrate S2 is preferably covered with a restricting member 11 into which the sample does not permeate. This is because, for example, a case where it is advantageous in filtration of interfering particles (described later) or a case where it is possible to reduce influence due to outside air by reducing an exposure area of a sample to outside air and, therefore, a measurement potential easily becomes stable.

The lower surface of the porous substrate S1 and the upper surface of the porous substrate S2 each are preferably covered with the restricting member 11.

### <Porous Substrate>

In the following Example, a paper material is used as a porous substrate; however, the material of the porous substrate is not limited to a paper material. The porous substrate may be made of any material that causes capillarity for liquid, may be a porous material of a mesh shaped structure or the like, such as open-cell bubbles and nanofibers, inside, or may be made of resin, glass, an inorganic board, cloth, metal paper, or the like. For example, a porous substrate having a large thickness or a high voidage is suitable for quick measurement because a sample flow rate due to capillarity is high.

In the following Example, the porous substrate S1 and the porous substrate S2 each are made of the same paper substrate one by one; however, the combination is not limited thereto. A channel region equivalent to that of this Example may be formed by folding the single porous substrate S1. The porous substrate S1 and the porous substrate S2 may be made of different materials. Depending on a combination of materials, the effect of flow rate control of a sample, filtration of a sample, or the like can be imparted to the device.

### <Channel Wall>

In the following Example, a hydrophobic resin is disposed and then a channel pattern (channel wall) is formed by heat fixing; however, a method of forming the channel pattern is not limited thereto. The method of forming a channel pattern just needs to form a channel pattern. Other than a method of cutting a paper porous substrate to leave only a channel shape, the method may be forming a channel wall with a wax printer.

### <Restricting Member>

The restricting member just needs to be made of a material into which a sample does not permeate.

Specifically, the restricting member may be a laminate film made of polyethylene terephthalate (PET).

The reference electrode 7 is disposed in the channel chamber 1 (first channel chamber). The upper surface and side surface of the reference electrode 7 are covered with the ionic crystal 10. The reference electrode 7 has a lead wire that the electrode continuously extends from the inside of the channel chamber 1 to above the channel wall 5 as a contact at the time of measurement.

The working electrode 8 is disposed in the channel chamber 2 (second channel chamber). The upper surface and side surface of the working electrode 8 are covered with the ion selective membrane 9 including a component having an ion selectivity. The working electrode 8 has a lead wire continuously extending from the inside of the channel chamber 2 to above the channel wall 5.

In the following Example, the channel chamber (first channel chamber) for the reference electrode 7 and the channel chamber (second channel chamber) for the working electrode 8 each are one by one; however, the number of channel chambers is not limited thereto. When the number of types of ions of which the concentration is intended to be measured is multiple, the working electrode and the channel chamber may be increased according to the number of the types of ions.

The porous substrate S2 includes the dispensing portion 6 and the channel 4. The dispensing portion 6 is disposed so as to overlap the channel 3 of the porous substrate S1. The channel 4 is disposed so as to overlap the channel 3 and the ion selective membrane 9. The channel 4 of the porous substrate S2 does not necessarily need to cover the entire upper surface of the working electrode 8. For example, in a working electrode having a good wettability for a sample, when the channel 4 covers part of the upper surface of the working electrode, a similar advantageous effect to that of the present embodiment is obtained.

### [Example 1] <Configuration of Channel>

In Example 1, the porous substrate S1 and the porous substrate S2 made of paper with a length of L1 = 0.1 mm and a voidage of 50% were used.

The voidage (%) was calculated by using the following formula. Voidage (%) = ((True density) - (Apparent density))/(True density) × 100

The apparent density (g/cm³) was calculated by using the following formula. (Apparent density) (g/cm3) = (Basis weight) (g/m2)/Thickness (mm) × 1000

After hydrophobic resin was disposed on the surface of the porous substrate S1, a channel pattern serving as the sample-impermeable channel wall 5 was formed by heat fixing. The porous substrate S2 was disposed so as to overlap the channel 3 and the ion selective membrane 9. The porous substrate S1 and the porous substrate S2 were covered with a restricting member into which a sample does not permeate. In this Example, a laminate film made of polyethylene terephthalate (PET) was used as the restricting member.

The sizes of the channel chambers 1, 2 and dispensing portion 6 were as follows.
Channel chamber 1: L11 = 6 mm, L12 = 6 mm
Channel chamber 2: L21 = 6 mm, L22 = 6 mm
Dispensing portion 6: a circle with a diameter of 3 mm Shortest distance L41 from the center of the dispensing portion 6 to the ionic crystal 10: 7 mm
Shortest distance L42 from the center of the dispensing portion 6 to the ion selective membrane 9: 6.5 mm Width L31 of the channel 3: 3 mm, Width L43 of the channel 4: 3 mm

### <Sample>

A sample was an aqueous solution containing NaCl that was measurement target ions and KCl that was coexisting ions.

### <Formulation of Electrodes>

Formulation of electrodes according to Example 1 will be described.

The reference electrode 7 made of Ag/AgCl was provided in the channel chamber 1. 3.5 mg of KCl ionic crystal was disposed on the reference electrode 7 as the ionic crystal 10.

The size of the reference electrode 7 was as follows. Reference electrode 7: L71 = 4 mm, L72 = 4 mm

On the other hand, the working electrode 8 mainly made of carbon as a main component was provided in the channel chamber 2. Instead of the carbon electrode, the working electrode 8 made of a conductive polymer, such as PEDOT:PSS (dispersions of polyethylene dioxythiophene and polystyrene sulfonate), may be used. A material like Ag/AgCl used for the base of the reference electrode in the existing art may be used.

The size of the working electrode 8 was as follows. Working electrode 8: L81 = 5.5 mm, L82 = 5.5 mm

An Na ion selective membrane 9 was formed so as to cover the working electrode 8. The ion selective membrane 9 was formed by the following materials.

| | |
|---|---|
| Ion selective material: Bis(12-crown-4) | 3.0mass% |
| Anion remover: Potassium tetrakis (4-chlorophenyl) borate | 0.5mass% |
| o-Nitrophenyl octyl ether | 64.0mass% |
| Polyvinyl chloride | 32.5mass% |

In this Example, the reference electrode 7, the working electrode 8, and the ion selective membrane 9 were formed with shapes, sizes, materials, and the like, as described above; however, the configuration is not limited thereto.

### <Ionic Crystal>

The ionic crystal 10 was formed so as to cover the reference electrode 7.

In this Example, a KCl ionic crystal was used as the ionic crystal 10. However, the material of the ionic crystal 10 just needs to include Cl ions and is not limited to a KCl ionic crystal. The mass of the ionic crystal 10 to be disposed falls within the range of the mass of KCl ionic crystal by which a saturated solution is obtained when the KCl ionic crystal is dissolved into pure water having a volume equivalent to the solution in the channel chamber 1, and is not limited thereto.

The ion selective membrane 9 was provided in this Example; however, in, for example, a case where the total amount of ions in a sample is measured, the ion selective membrane 9 does not necessarily need to be provided.

### <Permeation of Sample>

Permeation of a sample will be described.

A sample is dispensed by the dispensing portion 6 on the porous substrate S2, and then permeates through the paper porous substrates S1, S2 because of capillarity in accordance with a channel pattern at the same time in both the direction toward the channel chamber 1 and the direction toward the channel chamber 2.

While the dispensed sample permeates into the channel chamber 1, ions included in the sample are selected by the ion selective membrane 9 in the channel chamber 2, and the measurement potential of the working electrode 8 necessary to measure electrolyte concentration becomes stable.

In the present embodiment, the location of the dispensing portion 6 is a middle point at which a distance from the channel chamber 1 and a distance from the channel chamber 2 are substantially equal; however, the location of the dispensing portion 6 is not limited thereto. Depending on the location of the dispensing portion with respect to the ion selective membrane or the material of the porous substrate, it can be possible to improve sensitivity, selectivity, or the like by imparting a filtration function to the device for a sample including fine particles of about several micrometers in the sample.

### <Measurement of Sample Concentration>

Measurement of a sample concentration will be described.

Once the sample permeates and reaches the ionic crystal 10 covering the reference electrode 7, KCl ionic crystal dissolves in the sample, and the concentration of Cl ions in the solution in the channel chamber 1 saturates. At this time, when the measurement potential of the working electrode 8 is stable, measurement of the concentration of the sample is possible, and measurement of the sample concentration ends after a lapse of a predetermined measurement time. When the dispensed volume of the sample is greater than or equal to a volume by which the sample can reach the channel chamber 1 and the channel chamber 2 via the channel 3 of the porous substrate S2, it is possible to measure the sample concentration between the working electrode 8 and the reference electrode 7 with high accuracy by bringing a sufficient amount of the sample into contact with the ion selective membrane 9.

### [Comparative Example 1]

To describe the advantageous effect of Example 1 in more details, Comparative Example 1 will be described with reference to Fig. 3.

### <Configuration of Channel>

A microanalytical chip P2 includes the porous substrate S1 but does not include the porous substrate S2. In the microanalytical chip P2, the shapes of the porous substrate S1 and channel wall were the same as those of Example 1. The configuration (shape and size) of each of the dispensing portion 6, the reference electrode 7, the working electrode 8, the ion selective membrane 9, the ionic crystal 10, and the like was also the same as that of Example 1. However, the dispensing portion 6 was formed in the porous substrate S1. As described above, the porous substrate S2 was not used.

### <Permeation of Sample>

The dispensed sample began to permeate at the same time from the dispensing portion 6 in both the direction toward the channel chamber 1 and the direction toward the channel chamber 2 and reached the channel chamber 1 and the channel chamber 2 at the same time.

The sample having reached the channel chamber 1, as in the case of Example 1, dissolved the ionic crystal 10 covering the reference electrode 7 and formed a Cl ion solution having a saturated concentration.

On the other hand, the sample having reached the channel chamber 2 contacted with the ion selective membrane 9 but a part with which the sample mainly contacted was at a boundary location between the porous substrate S1 and the ion selective membrane 9 in the cross section of the porous substrate S1. The boundary location corresponds to a side surface part of the ion selective membrane 9 in Comparative Example 1. The sample did not contact with an upper surface part of the ion selective membrane 9. For this reason, there were cases where enough measurement sensitivity or ion selectivity was not obtained depending on the status of permeation of the ion selective membrane 9 for the voidage of the porous substrate S1 and the shape in which the ion selective membrane 9 was disposed in the porous substrate S1.

### [Advantageous Effect of Example 1]

### [Excellent Points of Example 1 over Comparative Example 1]

Three excellent points of Example 1 over Comparative Example 1 will be described.

### <Measurement of Sample Concentration>

Measurement was performed with a mixed solution method defined in JIS K0122 General rules for ion selective electrode method as an electrolyte concentration measurement method.

A solution containing measurement target ions was NaCl, and KCl was selected as a solution containing coexisting ions.

The concentration of KCl was fixed at 10 mmol/L, and then the concentration of NaCl was varied in five steps, that is, 100 µmol/L, 1 mmol/L, 10 mmol/L, 100 mmol/L, and 800 mmol/L. A sample dispensed amount was set to 30 µL to 50 µL. In relation to this measurement, to compare the advantageous effect of the porous substrate S2 in the working electrode 8, a commercially available silver-silver chloride electrode (RE-1BP, BAS Inc.) was used for the reference electrode side.

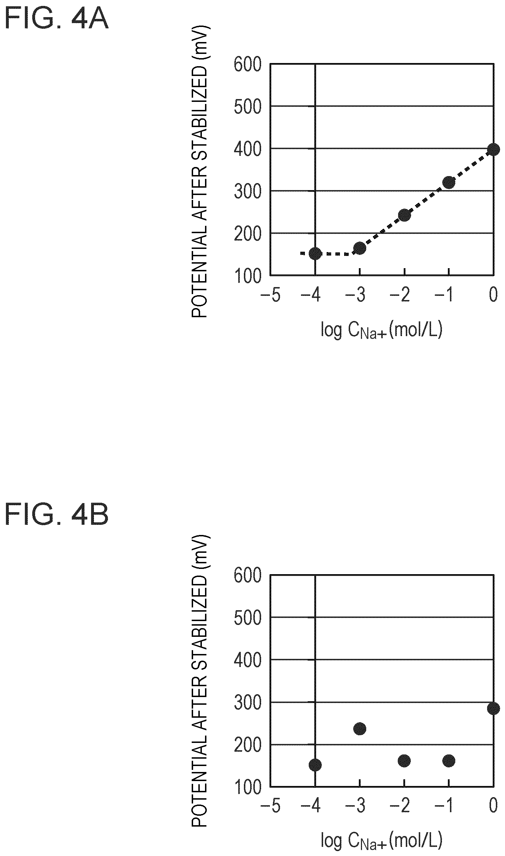

Figs. 4A and 4B show measurement results. Figs. 4A and 4B show the relationship between logarithmic Na ion concentration (logC_{Na}⁺) and measurement potential (potential after stabilized).

The sensitivity represents the slope of a region in which the slope of measurement potential to logarithmic Na ion concentration is constantly not negative. An ion selective coefficient represents that intended ions smaller by how many digits with respect to existing K ions can be detected and is desirable as increasing in a negative direction.

As shown in Fig. 4A, at a non-negative straight line rising point in Example 1, the logarithmic Na ion concentration was near -3 (mol/L). The logarithmic concentration of the coexisting ions in this Example is -2, so the graph represents that intended Na ions can be measured from a concentration smaller by one or more digits.

On the other hand, as shown in Fig. 4B, a region where the measurement potential rises was difficult to be determined in Comparative Example 1, so determination of the ion selective coefficient and sensitivity was also instable.

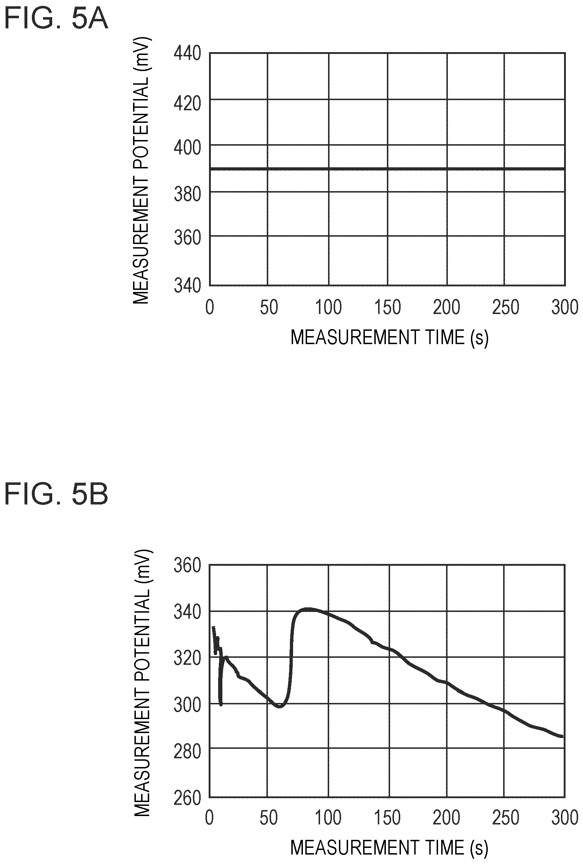

As shown in Figs. 5A and 5B, there is a difference in behavior between Example 1 and Comparative Example 1 also in a temporal stability of measurement potential. Fig. 5A shows a stable temporal change in potential in Example 1; whereas, Fig. 5B shows a steep change and a continuously moderate change in potential.

This demonstrated that a sample needed for measurement could be quickly and stably supplied to the ion selective membrane in Example 1, Example 1 was excellent in measurement sensitivity, ion selectivity, and temporal potential stability, and Example 1 was effective in electrolyte concentration measurement.

### <Filtration of Interfering Particles>

Filtration of interfering particles will be described.

In a case of a sample that includes particles that can interfere with electrolyte concentration measurement, it is desirable that interfering particles generally do not contact with the ion selective membrane in order to perform stable potential measurement.

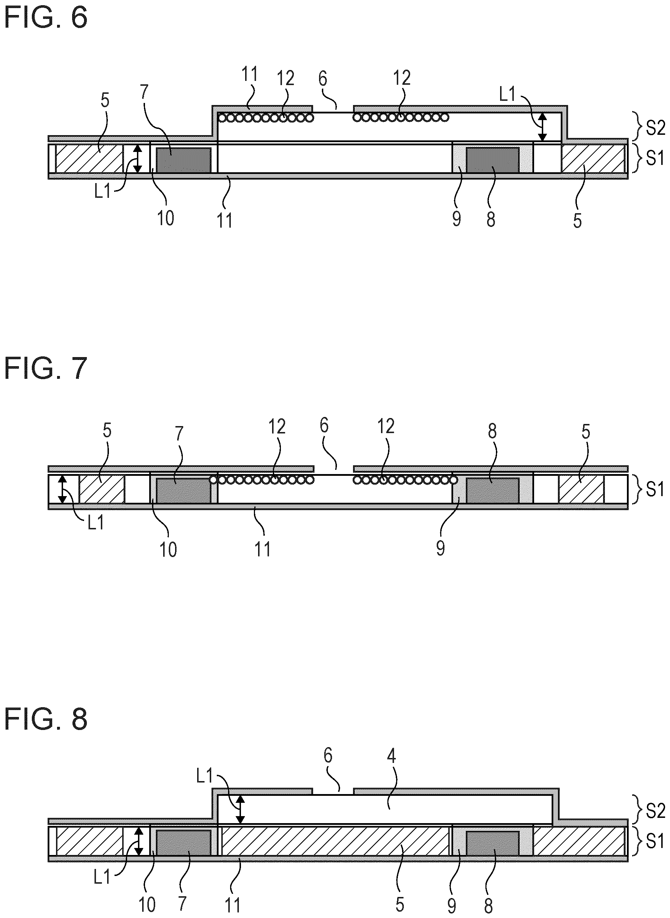

Fig. 6 shows a schematic diagram of a cross section in a case where 30 µL to 50 µL of a sample containing particles 61 of which styrene acrylic with about a number mean particle diameter of 8 µm is used as a main component for the microanalytical chip P1 used in Example 1. Fig. 7 shows a schematic diagram of a cross section in a case where 30 µL to 50 µL of a sample containing particles 61 of which styrene acrylic with about a number mean particle diameter of 8 µm is used as a main component for the microanalytical chip P2 used in Comparative Example 1. It was observed that the dispensed interfering particles (particles 61) stagnated near an interface between the porous substrate having the dispensing portion and the restricting member both in Example 1 and Comparative Example 1.

As shown in Fig. 6, in the microanalytical chip P1 of Example 1, interfering particles stagnate at the interface between the porous substrate S2 and the restricting member 11, so the interfering particles are difficult to be diffused to the inside of the porous substrate S1, so it is suitable as a configuration that reduces contact of interfering particles with the ion selective membrane 9.

On the other hand, as shown in Fig. 7, in the microanalytical chip P2 of Comparative Example 1, depending on a distance between the dispensing portion 6 and the ion selective membrane 9, interfering particles could reach the surface of the ion selective membrane 9 through permeation, so electrochemical measurement could be influenced.

For this reason, in measurement of a sample that contains particles that can interfere with electrochemical measurement, with the configuration like Example 1, it is possible to filtrate interfering particles without measurement preprocessing of a sample, so it is suitable as electrolyte concentration measurement.

### [Comparative Example 2]

Fig**.** 8 is a schematic sectional view that shows the configuration of a microanalytical chip P3 according to Comparative Example 2. The configuration of the microanalytical chip P3 shown in Fig**.** 8 simulates the configuration described in PTL 1.

### <Configuration of Channel>

The microanalytical chip P3 includes the porous substrate S1 and the porous substrate S2.

The porous substrate S1 is configured such that a region other than a region in which the reference electrode 7 and the working electrode 8 are formed is buried with the sample-impermeable channel wall 5.

The porous substrate S2 includes the dispensing portion 6 and the channel 4. The dispensing portion 6 is disposed substantially just above a middle point between the reference electrode 7 and the working electrode 8 of the porous substrate S1. The channel 4 is disposed so as to overlap the dispensing portion 6-side end of the ionic crystal 10 and the ion selective membrane 9 of the working electrode 8.

### <Reduction of Measurement Time>

A reduction of measurement time will be described.

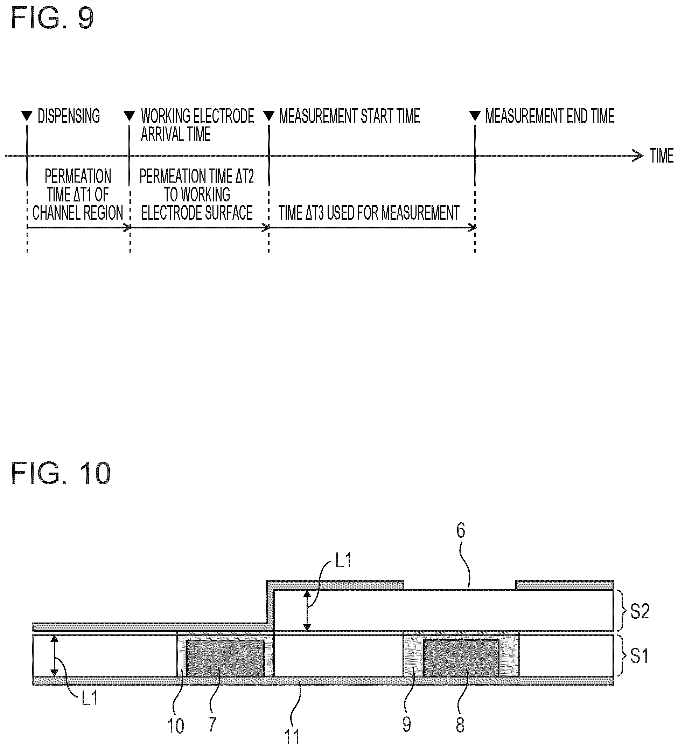

Fig. 9 shows a temporal change in the behavior of a sample after dispensing according to Example 1, Comparative Example 1, and Comparative Example 2.

ΔT1 denotes a period of time that the dispensed sample takes to fill a channel region by the time the dispensed sample reaches the working electrode, ΔT2 denotes a period of time that the sample having reached the working electrode takes to cover the surface of the working electrode to be ready to start measurement, and ΔT3 denotes a period of time that is taken from when electrochemical measurement starts to when the potential becomes stable and the measurement ends.

Table 1 shows temporal change results in the behavior of a sample in Example 1, Comparative Example 1, and Comparative Example 2. The results were measured at the time of the above-described five-step concentration measurement (the concentration of KCl was fixed at 10 mmol/L, and then the concentration of NaCl was varied in five steps, that is, 100 µmol/L, 1 mmol/L, 10 mmol/L, 100 mmol/L, and 800 mmol/L.), and an average was taken.

In Example 1, the channel volume can be increased by the amount by which a channel is formed in the porous substrate S2 and can increase the flow rate of a sample that permeates into the channel as compared to Comparative Example 1 or Comparative Example 2. This demonstrated that Example 1, as compared to Comparative Example 1 or Comparative Example 2, achieved a reduction of time in all the periods of time ΔT1, ΔT2, and ΔT3 and a total measurement time T (= ΔT1 + ΔT2 + ΔT3) could be reduced.

### [Table 1]

**Table 1**

| | Example 1 | Comparative Example 1 | Comparative Example 2 |
|---|---|---|---|
| ΔT1 [sec] | 1 | 9 | 4 |
| ΔT2 [sec] | 2 | 23 | 4 |
| ΔT3 [sec] | 120 | Unstable at 300 | 300 |

Table 2 summarizes excellent points of Example 1 over Comparative Example 1 and Comparative Example 2.

High sensitivity and ion selectivity were obtained from stable measurement potential over a lapse of time in Example 1. Sensitivity and ion selectivity were calculated in compliant with the above-described JIS K0122 General rules for ion selective electrode method.

On the other hand, this demonstrated that, in Comparative Example 1, ion selectivity in measurements of which the ion concentration was varied (the concentration of KCl was fixed at 10 mmol/L, and then the concentration of NaCl was varied in five steps, that is, 100 µmol/L, 1 mmol/L, 10 mmol/L, 100 mmol/L, and 800 mmol/L.) was not constant and concentration measurement could be instable.

In Comparative Example 2, a temporal change in potential was unstable and the total measurement time extended as compared to Example 1.

In the configuration of Example 1, while the area of the ion selective membrane 9 that contacts with the sample is increased, it is possible to quickly supply a sufficient amount of sample that reacts with the ion selective membrane 9 with high purity (Fig. 6). This demonstrated that the configuration of Example 1 was excellent in measurement sensitivity and ion selectivity (Figs. 4A and 4B) and temporal stability in measurement potential (Figs. 5A and 5B) and was effective in accurately measuring electrolyte concentration in a short time.

### [Table 2]

**Table 2**

| | Example 1 | Comparative Example 1 | Comparative Example 2 |
|---|---|---|---|
| Measurement Sensitivity | 86.2 | Unstable | 82.2 |
| Ion Selectivity | -0.97 | Unstable | -0.91 |
| Temporal Stability | Excellent | Not Good | Good |
| Filtration of Interfering Particles | Excellent | Not Good | Excellent |
| Total Measurement Time [sec] | About 120 | Not stable at 300 | 300 |

### [Modification 1 of Example 1]

A microanalytical chip P4 according to Modification 1 of Example 1 will be described.

Fig. 10 is a sectional view that shows the schematic configuration of the microanalytical chip P4. In this Modification, the dispensing portion 6 is disposed at the upper surface (substantially just above) the working electrode 8.

Thus, it is possible to reduce a distance between the reference electrode 7 and the working electrode 8 while having high measurement performance, such as sensitivity, ion selectivity, and temporal stability), with the result that the device can be miniaturized.

The present invention is not limited to the above-described embodiments. Various changes or modifications are applicable without departing from the spirit and scope of the present invention. Therefore, the following claims are attached to show the scope of the present invention.

This application claims the benefit of Japanese Patent Application No. 2022-071207 filed April 23, 2022, which is hereby incorporated by reference herein in its entirety.

## Claims

1. A microanalytical chip made up of a first porous substrate and a second porous substrate laminated on each other, wherein
a first channel chamber, a second channel chamber, and a first channel connecting the first channel chamber with the second channel chamber are formed by a channel wall in the first porous substrate,
a reference electrode is disposed in the first channel chamber,
a working electrode is disposed in the second channel chamber, and
the second porous substrate is disposed so as to overlap at least part of a region, where the working electrode is formed, on a surface of the first porous substrate.

2. The microanalytical chip according to Claim **1,** wherein the working electrode is covered with an ion selective membrane including a component having an ion selectivity.

3. The microanalytical chip according to Claim **1,** wherein the second porous substrate has a dispensing portion for dispensing a sample.

4. The microanalytical chip according to Claim 1 or 2, further comprising a restricting member into which a sample does not permeate, the restricting member being disposed at least at a location where the dispensing portion and the working electrode are connected, on an upper surface of the second porous substrate.

5. The microanalytical chip according to Claim 1, further comprising an ionic crystal having a sample solubility, the ionic crystal being disposed on a surface of the reference electrode.
